(19) Europäisches Patentamt
European Patent Office
Office européen des brevets

(11) **EP 3 318 235 A1**

(12) **EUROPEAN PATENT APPLICATION**
published in accordance with Art. 153(4) EPC

(43) Date of publication:
**09.05.2018 Bulletin 2018/19**

(51) Int Cl.:
***A61F 13/511*** *(2006.01)*

(21) Application number: **16817594.1**

(22) Date of filing: **23.05.2016**

(86) International application number:
**PCT/JP2016/065207**

(87) International publication number:
**WO 2017/002485 (05.01.2017 Gazette 2017/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **30.06.2015 JP 2015132213**

(71) Applicant: **Unicharm Corporation**
**Ehime 799-0111 (JP)**

(72) Inventors:
• **TAKEDA, Eisuke**
**Kanonji-shi**
**Kagawa 769-1602 (JP)**
• **FUNABA, Maika**
**Kanonji-shi**
**Kagawa 769-1602 (JP)**
• **IWAI, Wakana**
**Kanonji-shi**
**Kagawa 769-1602 (JP)**

(74) Representative: **Peter, Julian**
**Staeger & Sperling**
**Partnerschaftsgesellschaft mbB**
**Sonnenstrasse 19**
**80331 München (DE)**

(54) **SURFACE SHEET FOR ABSORBENT ARTICLE AND ABSORBENT ARTICLE USING SAME**

(57) The present invention relates to a surface sheet for absorbent articles, and provides a surface sheet which is excellent in liquid absorption rate and in feeling of wear when being used for an absorbent article and which is excellent in liquid drainage. The surface sheet (2) for absorbent articles according to the present invention includes: a nonwoven fabric made of a short fiber; and a hydrophobic granular gel composition (G) attached to the short fiber, and has a dispersion region (6) in a skin-facing-side surface (21) of the surface sheet (2), in which the granular gel composition (G) is present in a dispersed state, and in the dispersion region (6), a dispersion state coefficient (V) representing the dispersion state of the granular gel composition (G) is 900-5000.

FIG. 1

**Description**

Technical Field

The present invention relates to a top sheet for an absorbent article that includes a gel composition, and to an absorbent article using the same.

Background Art

In recent years, absorbent articles such as disposable diapers and sanitary napkins have been proposed wherein lotions containing skin care components or the like are coated on their top sheets, for the purpose of obtaining a skin care effect or a comfortable feel during wear. Such an absorbent article is disclosed in PTL 1, for example, as a disposable absorbent article having a lotion added in a multi-strip pattern on at least a portion of the skin side surface of the top sheet. With the disposable absorbent article disclosed in PTL 1, a comfortable feel during wear and an excellent liquid treatment property and smooth feel are obtained.

Also, PTL 2 discloses an absorbent article that can maintain a gel state at 38°C on the skin side surface of the top sheet, and that is intermittently coated with a hydrophobic gel composition comprising a styrene-based thermoplastic elastomer which is a triblock or greater block copolymer including a polystyrene-based hard segment and a soft segment, and a hydrocarbon oil. The absorbent article disclosed in PTL 2 has excellent dryness on the surface and an excellent liquid permeation property in the thickness direction, while also maintaining the bonded state between the top sheet and core wrap sheet and having an excellent absorption property.

Citation List

Patent Literature

  [PTL 1] Japanese Patent Public Inspection No. 2008-522772
  [PTL 2] Japanese Patent Publication No. 5677611

Summary of Invention

Technical Problem

The top sheets used in the absorbent articles disclosed in the aforementioned patent literature, while exhibiting excellent feel during wear and a high liquid absorption rate, have been in need of further improvement in terms of the liquid repellent property (liquid repelling rate).

It is therefore an object of the present invention to provide a top sheet which is a top sheet for an absorbent article having excellent feel during wear and a high liquid absorption rate when used in an absorbent article, while also having an excellent liquid repellent property.

Solution to Problem

One aspect (aspect 1) of the invention is a top sheet for an absorbent article wherein the top sheet comprises a nonwoven fabric composed of staple fibers and a hydrophobic granular gel composition adhering to the staple fibers, and a skin side surface of the top sheet has a dispersing region in which the granular gel composition is present in a dispersed state, a dispersed state coefficient (V), which represents the dispersed state of the granular gel composition, being 900 to 5000 in the dispersing region.

According to the top sheet of aspect 1, since the granular gel composition is present on the skin side surface of the top sheet in a dispersed state with a dispersed state coefficient (V) of 900 to 5000, synergistic action between the hydrophobic action of the gel composition that has a hydrophobic property (i.e., the action of pushing excreted fluid such as urine into the top sheet) and the water-absorbing action of the staple fibers with a hydrophilic property inside the top sheet (i.e., the action of drawing in the excreted fluid such as urine into the top sheet) allows the excreted fluid such as urine supplied to the skin side surface of the top sheet to form a state in which it is easily drawn into the top sheet through the sections where the gel composition is not present, thereby allowing an excellent absorption rate and liquid repellent property (liquid repelling rate) to be obtained for the top sheet. As a result, the top sheet of aspect 1 can provide an excellent smooth feel during wear when used in an absorbent article.

In addition, since the top sheet of aspect 1 comprises a nonwoven fabric composed of staple fibers and a hydrophobic granular gel composition adhering to the staple fibers, the granular gel composition easily infiltrates between the staple fibers composing the nonwoven fabric, and in the dispersing region mentioned above, some of the granular gel composition infiltrates to the interior of the top sheet. Moreover, since excreted fluid such as urine tends to be pushed even deeper than the interior of the top sheet (to the non-skin side), due to the hydrophobic action of the granular gel composition that has infiltrated the interior, the aforementioned excellent absorption rate and liquid repellent property (liquid repelling rate) can be even more effectively exhibited.

According to the invention, the dispersed state coefficient (V) is the value representing the dispersed state of the granular gel composition, determined by multiplying the variation in the granular gel composition present in a dispersed state in the dispersing region of the gel composition, by the degree of aggregation of the granular gel composition (the black value described below), and the specific method for measuring this value will be described below.

According to another aspect (aspect 2) of the invention, in the top sheet of aspect 1, the dispersed state coefficient (V) of the top sheet in the region corresponding to the dispersing region on the non-skin side surface is 4000 to 6000.

If the dispersed state coefficient (V) on the non-skin side surface of the top sheet is 4000 to 6000, then some of the granular gel composition in the dispersing region on the skin side surface of the top sheet will infiltrate to the depth or to the non-skin side surface (hereunder referred to as "depth and further"), and the granular gel composition that has infiltrated to the depth and further will be dispersed in the depth and further part of the top sheet in a dispersed state with a dispersed state coefficient (V) of 4000 to 6000 on the non-skin side surface of the top sheet, allowing formation of a state in which excreted fluid such as urine is easily drawn in even deeper than the interior of the top sheet (the non-skin side) or to the non-skin side surface. Therefore, the top sheet of aspect 2 can more effectively exhibit the function and effect of the top sheet of aspect 1 (especially the liquid repellent property (liquid repelling rate)).

According to yet another aspect (aspect 3) of the invention, in the top sheet of aspect 1 or 2, the gel composition has a specific viscosity characteristic with a viscosity of 100 to 500 mPa·s at 37°C.

Since the gel composition can be coated at relatively low temperature when the gel composition has this specific viscosity characteristic, the components in the gel composition are less likely to undergo deterioration or volatilization (i.e., the composition of the gel composition is less likely to vary), and the function and effect of the gel composition can be stably exhibited. In addition, such a gel composition easily maintains its state immediately after coating, and any changes in the dispersed state coefficient (V), i.e. changes in the dispersed state (especially changes in the size of the granular gel composition) that accompany temperature changes after coating are less likely to occur.

Consequently, the top sheet of aspect 3 can more reliably exhibit the aforementioned function and effect by which an excellent absorption rate and liquid repellent property can be obtained.

According to yet another aspect (aspect 4) of the invention, in the top sheet of any one of aspects 1 to 3, the gel composition is present at a basis weight of 1 to 50 g/m$^2$ in the dispersing region.

If the basis weight of the gel composition is 1 g/m$^2$ or greater, the hydrophobic action on the skin side surface of the top sheet (the action of pushing in excreted fluid) will be adequately exhibited, and therefore excreted fluid such as urine can be pushed into the top sheet in a steady manner and the absorption rate and liquid repellent property can be improved in a steady manner. Furthermore, if the basis weight of the gel composition is 50 g/m$^2$ or lower, excreted fluid supplied to the skin side surface of the top sheet will not be repelled by the hydrophobic action of the gel composition but will be able to be stably pushed into the top sheet, and therefore the absorption rate and liquid repellent property can be exhibited in an even more steady manner.

According to yet another aspect (aspect 5) of the invention, in the top sheet of any one of aspects 1 to 4, the gel composition is a composition comprising a styrene-based thermoplastic elastomer that forms a gel network structure, and a hydrocarbon oil.

Since the gel composition in the top sheet of aspect 5 is a composition comprising a styrene-based thermoplastic elastomer that forms a gel network structure and a hydrocarbon oil, the feel on the skin of the top sheet and the feel during wear of the absorbent article using the top sheet can be further improved, in addition to the aforementioned function and effect of improving the absorption rate and liquid repellent property, due to the properties of the composition, i.e. the properties of having suitable flexibility while being unlikely to produce tackiness or a sticky feel. In addition, since such a gel composition can maintain its gel state even under temperature conditions of 38°C, the gel state is maintained and the aforementioned properties can be stably exhibited even when the temperature of the gel composition has increased by body heat of the wearer or the like.

According to yet another aspect (aspect 6) of the invention, in the top sheet of aspect 5, the styrene-based thermoplastic elastomer is a styrene-based thermoplastic elastomer composed of a diblock copolymer.

A styrene-based thermoplastic elastomer composed of a diblock copolymer has less or weaker aggregation between the styrene hard segments compared to a styrene-based thermoplastic elastomer composed of a triblock or greater block copolymer of the same molecular weight, while the gel network structure formed by the styrene-based thermoplastic elastomer is not firm, and therefore the gel composition easily flows even a relatively low temperature, the state immediately after coating can be easily maintained, and changes in the dispersed state coefficient (V) that accompany temperature changes after coating, or in other words changes in the dispersed state (especially changes in the size of the granular gel composition), are less likely to occur. As a result, the top sheet of aspect 6 can more stably and reliably exhibit the function and effect by which an excellent absorption rate and liquid repellent property can be obtained.

According to yet another aspect (aspect 7) of the invention, in the top sheet of any one of aspects 1 to 6, the gel composition is a composition comprising 100 parts by mass of styrene-based thermoplastic elastomer (A) composed of a diblock copolymer with a weight-average molecular weight of from 100,000 to 200,000, and 500 to 4800 parts by mass of a hydrocarbon oil (B) with a kinematic viscosity of 5 to 50 $mm^2/s$ at 37.8°C.

Since the gel composition of the top sheet of aspect 7 is a composition comprising a styrene-based thermoplastic elastomer (A) composed of a diblock copolymer with a specific molecular weight and a hydrocarbon oil (B) with a specific kinematic viscosity, in a prescribed ratio, the function and effect of the top sheet of aspects 1 to 6 described above, and especially the function and effect of the top sheets of aspects 5 and 6, can be more effectively and more reliably exhibited.

Yet another aspect (aspect 8) of the invention is an absorbent article including a top sheet according to any one of aspects 1 to 7.

In the absorbent article of aspect 8, the top sheet exhibits the function and effect of the top sheet of any of aspects 1 to 7, and therefore the absorbent article can exhibit an excellent feel during wear, and also an excellent absorption rate and liquid repellent property (liquid repelling rate) for excreted fluid such as urine.

Advantageous Effects of Invention

According to the invention it is possible to provide a top sheet which is a top sheet for an absorbent article having excellent feel during wear and a high liquid absorption rate when used in an absorbent article, while also having an excellent liquid repellent property.

Brief Description of the Drawings

Fig. 1 is a plan view of the extended state of an absorbent article that employs a top sheet according an embodiment of the invention.
Fig. 2 is a partial magnified view of a cross-section of the absorbent article of Fig. 1 along line II-II' .

Description of Embodiments

The top sheet of the invention will now be explained in greater detail with reference to the accompanying drawings.

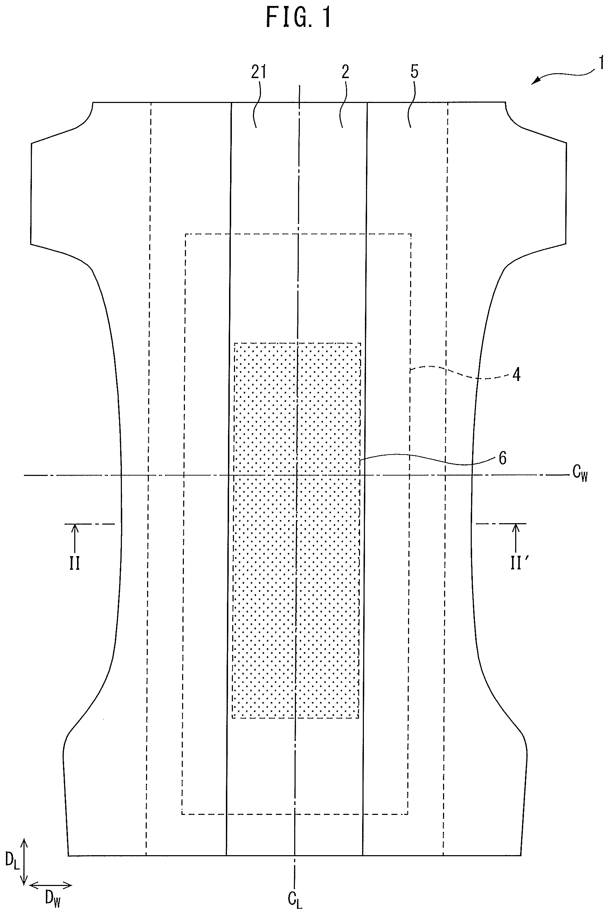

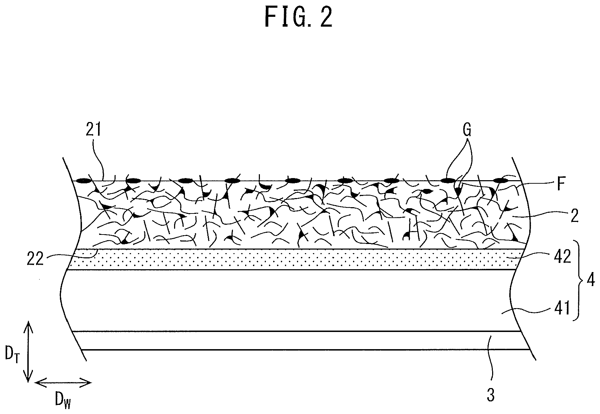

Fig. 1 is a plan view of the extended state of an absorbent article (disposable diaper 1) that employs a top sheet 2 according to an embodiment of the invention, and Fig. 2 is a partial magnified view (schematic view) of a cross-section of the absorbent article of Fig. 1 along line II-II'.

The disposable diaper 1 that employs a top sheet 2 according to this embodiment of the invention, in the planar view in the extended state, has an outer shape that is long in the lengthwise direction $D_L$, the approximate center section in the lengthwise direction $D_L$ having an essentially narrow-neck hourglass shape toward the interior in the widthwise direction $D_W$. Incidentally, the absorbent article to which the top sheet of the invention is applied is not limited to such an outer shape, and any longitudinal shape (for example, rectangular, elliptical, gourd-shaped or the like) may be used depending on the purpose of use, so long as it is elongated, with a length dimension in the lengthwise direction $D_L$ that is longer than the width dimension in the widthwise direction $D_W$.

Throughout the present description, unless otherwise specified, the simple phrase "planar view" will be used to mean the view of an object in the extended flat state from the upper side in the thickness direction.

Also throughout the present description, for the widthwise direction $D_W$ of the longitudinal absorbent article, the direction toward the center axis line $C_L$ running in the lengthwise direction $D_L$ will be referred to as the "inner side direction in the widthwise direction", and the direction away from the center axis line $C_L$ will be referred to as the "outer side direction in the widthwise direction". Similarly, for the lengthwise direction $D_L$ of the longitudinal absorbent article, the direction toward the center axis line $C_W$ running in the widthwise direction $D_W$ will be referred to as the "inner side direction in the lengthwise direction", and the direction away from the center axis line $C_W$ will be referred to as the "outer side direction in the lengthwise direction".

Moreover, for the thickness direction $D_T$ of the absorbent article, the direction toward the side facing the skin surface of the wearer (the skin side) will be referred to as the skin side direction, and the direction toward the side opposite the side facing the skin surface of the wearer (the non-skin side) will be referred to as the non-skin side direction.

As shown in Fig. 1 and Fig. 2, the disposable diaper 1 has a layered structure comprising a top sheet 2 situated on the skin side of the wearer in the thickness direction $D_T$, a liquid-impermeable back sheet 3 situated on the side opposite the skin side (i.e. the non-skin side), and an absorbent body 4 positioned between these sheets, and also a pair of side sheets 5 for formation of gather sections, situated on the skin side of the top sheet 2 with the top sheet 2 sandwiched between them on its outer side in the widthwise direction $D_W$ in the planar view. Also, on the skin side surface 21 of the top sheet 2, there is a dispersing region 6, which is a region coated with a hydrophobic gel composition described below, in which a fine granular gel composition G is present in a dispersed state. For this embodiment, as shown in Fig. 1, the dispersing region 6 is a rectangular region straddling both the center axis line $C_L$ running in the lengthwise direction $D_L$ and the center axis line $C_W$ running in the widthwise direction $D_W$ of the disposable diaper 1, in the planar view, and extending in both the widthwise direction $D_W$ and the lengthwise direction $D_L$; however, the top sheet of the invention is not limited to this aspect, and the dispersing region may be situated at any location of the top sheet, and its shape in the planar view may be any desired shape other than rectangular (including regular shapes such as circular, elliptical or triangular, and also including irregular shapes).

The top sheet 2 of this embodiment has the granular gel composition G present on the skin side surface 21 of the top sheet 2 in a dispersed state such that the dispersed state coefficient (V) is in the range of 900 to 5000 in the dispersing region 6. When the granular gel composition G is present in this range manner, synergistic action between the hydrophobic action of the gel composition that has a hydrophobic property (i.e., the action of pushing excreted fluid such as urine into the top sheet 2) and the water-absorbing action of the staple fibers F with a hydrophilic property (i.e., the action of drawing in the excreted fluid such as urine into the top sheet 2) inside the top sheet allows the excreted fluid such as urine supplied to the skin side surface 21 of the top sheet 2 to form a state in which it is easily drawn into the top sheet 2 through the sections where the granular gel composition G is not present, thereby allowing an excellent absorption rate and liquid repellent property (liquid repelling rate) to be obtained for the top sheet. As a result, the disposable diaper 1 using the top sheet 2 can provide the wearer with an excellent smooth feel during wear. The aforementioned synergistic action is difficult to obtain with a dispersed state in which the dispersed state coefficient (V) is outside of the range of 900 to 5000. Furthermore, in the top sheet of the invention, the dispersed state coefficient (V) representing the dispersed state of the granular gel composition is preferably in the range of 900 to 3000 and more preferably in the range of 900 to 2000 from the viewpoint of obtaining a more excellent liquid repellent property.

According to the invention, the dispersed state coefficient (V) representing the dispersed state of the granular gel composition may be determined by multiplying the variation in the granular gel composition present in a dispersed state in the dispersing region of the gel composition, by the degree of aggregation of the granular gel composition (the black value described below). Specifically, the dispersed state coefficient (V) may be determined by the following method of measurement.

[Method of measuring dispersed state coefficient (V)]

(1) A 10 g portion of black toner (replacement toner for laser printer, product of Canon Inc.) is sprinkled in an evenly distributed manner on the surface of a base material (nonwoven fabric) coated with the gel composition (hereunder referred to as "measuring surface"), and the black toner is adhered onto the gel composition.

(2) The measuring surface is blasted with air at 0.3 MPa from a location 200 mm away, to blow off only the black toner that is not adhering to the gel composition and remove it from the measuring surface. This allows the gel composition that has been blackened by adhesion of the black toner dispersed on the measuring surface to be visibly discerned.

(3) Next, the measuring surface on which the blackened gel composition has dispersed is imaged with at 20x magnification using a microscope (VHX-2000 microscope by Keyence Corp.), and the image data is subjected to "line color composition analysis" using image analysis software (USB Digital Scale by Scalar Corp.).

(4) In the line color composition analysis, the color composition on an arbitrary line on the measuring surface on which the blackened gel composition has dispersed is digitized to numerical values in the RGB color system. The average value for the numerical data for red, blue and green (i.e., $(R + G + B)/3$) and the standard deviation is calculated. According to the invention, the calculated value of the standard deviation is referred to as the measured "variation", and the average value is referred to as the measured "black value".

The "variation" represents the degree of uniformity of dispersion of the granular gel composition, a lower value indicating greater uniformity. The "black value" is the digitized representation of the area of the blackened gel composition and the level of blackness, and it represents the degree of aggregation of the granular gel composition.

(5) By then multiplying the "variation" and the "black value" obtained as described above, it is possible to obtain the dispersed state coefficient (V), representing the dispersed state of the granular gel composition on the measuring surface.

In the top sheet 2 of this embodiment, the dispersed state coefficient (V) in the region corresponding to the dispersing region 6 on the non-skin side surface 22 is 4000 to 6000. If the dispersed state coefficient (V) on the non-skin side surface of the top sheet is in the range of 4000 to 6000 as according to this embodiment, then some of the granular gel composition in the dispersing region on the skin side surface of the top sheet infiltrates deeper, and the granular gel composition that has infiltrated deeper and further is dispersed to the deeper and further part of the top sheet in a dispersed state with a dispersed state coefficient (V) in the range of 4000 to 6000 on the non-skin side surface of the top sheet, allowing formation of a state in which excreted fluid such as urine is easily drawn in even deeper than the interior of the top sheet (the non-skin side) or to the non-skin side surface. The top sheet 2 of this embodiment can therefore exhibit a particularly excellent liquid repellent property (liquid repelling rate). In the top sheet 2 of this embodiment, the dispersed state coefficient (V) in the region corresponding to the dispersing region 6 on the non-skin side surface 22 is in the range of 4000 to 6000, and preferably in the range of 4100 to 5000.

The dispersed state coefficient (V) on the non-skin side surface of the top sheet can be determined according [Method of measuring dispersed state coefficient (V)] above, with the non-skin side surface of the top sheet as the measuring surface.

Incidentally, throughout the present description, the term "absorption rate" means the rate of absorption of excreted fluid supplied onto the skin side surface of the top sheet from the skin side surface when it has been absorbed into the top sheet, and it can be quantified and evaluated based on the time required for the excreted fluid to migrate into the top sheet from the skin side surface of the top sheet. Also throughout the present description, the term "liquid repellent property" means the ease with which excreted fluid supplied onto the skin side surface of the top sheet is repelled when it is repelled from the skin side surface to the non-skin side, and it can be quantified and evaluated based on the time required for excreted fluid to be repelled from the skin side surface of the top sheet to the non-skin side, and be removed from the top sheet (the excreted fluid passing through the top sheet and completing migration to the absorbent body side) (i.e. the "liquid repelling rate").

The absorption rate and liquid repelling rate can be measured by the following absorption property evaluation test.

[Absorption property evaluation test]

(1) The top sheet is removed from a commercially available infant paper diaper (Moony "Air-Fit" S-size, by Unicharm Corp.), and the top sheet to be evaluated (i.e., a gel composition-coated top sheet) is attached to the section where the top sheet was removed, to produce a sample for absorption property evaluation testing.

(2) The sample for absorption property evaluation testing is attached onto a flat acrylic board and extended to the product length.

(3) A mark is made at the location to be evaluated on the top sheet of the sample for absorption property evaluation testing (the location where artificial urine is to be dropped).

(4) A cylinder is placed on the top sheet of the sample for absorption property evaluation testing with the mark located at the center.

(5) A burette containing artificial urine is situated inside the cylinder so that the tip of the burette is positioned 10 mm below the top end of the cylinder.

(6) The burette is used to drop 40 ml of artificial urine once onto the top sheet of the sample for absorption property evaluation testing, the time (sec) from initial dropping of the artificial urine until all of the artificial urine has completely migrated into the top sheet (until the artificial urine in the cylinder disappears) is measured, and the measured time is recorded as the absorption rate (sec).

(7) Also, the time (sec) from initial dropping of the artificial urine until all of the artificial urine has completely migrated through the top sheet to the absorbent body side is measured, and the measured time is recorded as the liquid repelling rate (sec).

(8) After 5 minutes has elapsed from initial dropping of the artificial urine, a second dropping of artificial urine is initiated, and the measurements of (6) and (7) are performed.

(9) After 5 minutes has elapsed from the second dropping of the artificial urine, a third dropping of artificial urine is initiated, and the measurements of (6) and (7) are performed.

(10) The absorption rate (sec) and liquid repelling rate (sec) obtained by the third measurement are used as the absorption rate (sec) and liquid repelling rate (sec), respectively, for the sample for absorption property evaluation testing.

The artificial urine is prepared by dissolving 200 g of urea, 80 g of sodium chloride, 8 g of magnesium sulfate, 3 g of calcium chloride and approximately 1 g of dye (Blue #1) in 10 L of ion-exchanged water.

The gel composition to be used in the top sheet of the invention will now be explained in detail.

[Gel composition]

In the top sheet of the invention, the gel composition to be coated onto the skin side surface of the top sheet may be any gel composition without any particular restrictions, so long as it is a hydrophobic gel composition that can be dispersed in a prescribed dispersed state such that the dispersed state coefficient (V) is in the range of 900 to 5000, using any coating means such as spray coating. Examples of such gel compositions include compositions comprising styrene-based thermoplastic elastomers that form a gel network structure, among which the aforementioned composition comprising a styrene-based thermoplastic elastomer and a hydrocarbon oil is preferably used. When a composition comprising a styrene-based thermoplastic elastomer and a hydrocarbon oil is used as the gel composition, the properties of the composition, namely the suitable flexibility imparted by the styrene-based thermoplastic elastomer, together with the property of helping to prevent a tacky or sticky feel, which is imparted by the hydrocarbon oil, can further improve the skin feel of the top sheet and the feel during wear of the absorbent article using the top sheet. In addition, since such a gel composition can maintain its gel state even under temperature conditions of 38°C, the gel state is maintained and the aforementioned properties can be stably exhibited even when the temperature of the gel composition has increased by body heat of the wearer or the like.

The styrene-based thermoplastic elastomer to be used in the gel composition is not particularly restricted so long as it can form a gel network structure, and examples include diblock copolymers, or triblock or greater block copolymers, that include a polystyrene-based hard segment and any desired soft segment. There are no particular restrictions on the polystyrene-based hard segment, and for example, it may be a polystyrene-based polymer such as polystyrene, poly(a-methylstyrene), poly(o-methylstyrene), poly(m-methylstyrene) or poly(p-methylstyrene). There are also no particular restrictions on the soft segment, and for example, it may be a polyolefin-based polymer such as polyethylene, polypropylene, polybutylene, polybutadiene or polyisoprene.

Also, the block copolymer includes preferably 10 to 50 mass% of a styrene-based block component, and 50 to 90 mass% of an olefin-based block component, more preferably 15 to 40 mass% of a styrene-based block component and 60 to 85 mass% of an olefin-based block component, and even more preferably 18 to 35 mass% of a styrene-based block component and 65 to 82 mass% of an olefin-based block component. If the proportion of the styrene-based block component is 10 mass% or greater, a fixed minimum number of aggregate domains formed by interaction with the polystyrene-based hard segment can be ensured, and therefore the styrene-based thermoplastic elastomer will be more likely to form a mesh-like network structure. Also, if the proportion of the styrene-based block component is 50 mass% or lower, it will be possible to ensure a fixed minimum number of olefin-based block components that hold the lubricant, described below, and therefore more of the lubricant can be held in the mesh-like network structure. Since the number of aggregate domains formed by the polystyrene-based hard segment is kept below a fixed minimum, this avoids hardening of the gel after its application onto the top sheet, and any resulting discomfort or unpleasantness caused for the wearer when the absorbent article is worn.

A diblock copolymer to be used as the styrene-based thermoplastic elastomer is not particularly restricted and may be a styrene-ethylene/propylene block copolymer (SEP), styrene-butadiene block copolymer (SB), styrene-isobutylene block copolymer or the like, and any of these block copolymers may be used either alone or in desired combinations of two or more. Also, triblock or greater block copolymers that may be used as the styrene-based thermoplastic elastomer are not particularly restricted and examples include styrene-butadiene-styrene block copolymer (SBS), styrene-ethylene-butylene-styrene block copolymer (SEBS), styrene-isoprene-butylene-styrene block copolymer (SIBS), styrene-isoprene-styrene block copolymer (SIS), styrene-ethylene-propylene-styrene block copolymer (SEPS), styrene-ethylene-ethylene-propylene-styrene block copolymer (SEEPS), with any of these block copolymers being used either alone or in desired combinations of two or more.

Since styrene-based thermoplastic elastomers comprising diblock copolymers and triblock or greater block copolymers have a mesh-like network structure formed by a plurality of aggregate domains created by interaction of the polystyrene-based hard segments, and soft segments linked to the plurality of aggregate domains, they exhibit functions as elastic solids, such as flexibility, while being able to stably maintain a gel state even under temperature conditions of body temperature (about 35°C to about 38°C). In addition, because such a mesh-like network structure releases the lubricant (for example, a hydrocarbon oil or silicone oil) in a suitable amount while also having the function of retaining it, the function of the lubricant can be stably exhibited for prolonged periods.

According to the invention, the styrene-based thermoplastic elastomer to be used in the gel composition is preferably a styrene-based thermoplastic elastomer composed of any of the aforementioned block copolymers, and particularly a diblock copolymer. A styrene-based thermoplastic elastomer composed of a diblock copolymer has less or weaker aggregation between the polystyrene-based hard segments compared to a styrene-based thermoplastic elastomer composed of a triblock or greater block copolymer of the same molecular weight, while the gel network structure is not firm, and therefore the gel composition easily flows even at relatively low temperature, the state immediately after coating can be easily maintained, and changes in the dispersed state coefficient (V) that accompany temperature changes after coating, or in other words changes in the dispersed state (especially changes in the size of the granular gel composition), are less likely to occur. As a result, the function and effect of obtaining an excellent absorption rate and liquid repellent property can be exhibited more stably and reliably.

According to the invention, the gel composition used is most preferably a composition comprising 100 parts by mass of styrene-based thermoplastic elastomer (A) composed of a diblock copolymer with a weight-average molecular weight of from 100,000 to 200,000, and 500 to 4800 parts by mass of a hydrocarbon oil (B) with a kinematic viscosity of 5 to 50 mm$^2$/s at 37.8°C. Such a composition is particularly superior in terms of the retentivity of the gel state after coating onto the top sheet (especially the retentivity at 38°C), as well as the gel hardness and elongation, when it is used as the gel composition of the invention, while the function and effect of obtaining an excellent absorption rate and liquid repellent property as described above can be exhibited even more stably, and the feel during wear of the absorbent article can be even more improved. Incidentally, the styrene-based thermoplastic elastomer (A) may also be a mixture of two or more styrene-based thermoplastic elastomers with different weight-average molecular weights, so long as the weight-average molecular weight is in the range of from 100,000 to 200, 000.

The weight-average molecular weight (Mw) of the styrene-based thermoplastic elastomer can be determined in terms of polystyrene, by GPC measurement under the following conditions using tetrahydrofuran (THF) as the mobile phase.

[GPC measuring conditions]

Apparatus: GPC-8220 (product of Tosoh Corp.)
Column: SHODEX KF-804 (product of Showa Denko K.K.)
Temperature: 40°C.
Solvent: THF
Flow rate: 1.0 mL/min
Sample concentration: 0.05 to 0.6 mass%
Injection rate: 0.1 mL
Detection: RI (differential refractometer)

The hydrocarbon oil (B) is not particularly restricted so long as it is a compound comprising carbon and hydrogen, with a kinematic viscosity of 5 to 50 mm$^2$/s at 37.8°C, and it may have a straight-chain, branched or cyclic structure, and have either saturated or unsaturated bonds. Examples for the hydrocarbon oil (B) include olefin-based hydrocarbons, paraffinic hydrocarbons, acetylene-based hydrocarbons, hydrocarbons including two or more double bonds and/or triple bonds, or cyclic hydrocarbons such as aromatic hydrocarbons or alicyclic hydrocarbons. More specifically, there may be mentioned hydrogenated polyisobutene, liquid paraffin, squalane and squalene, among which hydrogenated polyisobutene is particularly preferred for use because it does not produce tack in the gel after application onto the top sheet, and also does not produce stickiness by the controlled-release lubricant.

The hydrocarbon oil (B) has a 37.8°C kinematic viscosity in the range of 5 to 50 mm$^2$/s, preferably in the range of 10 to 30 mm$^2$/s and more preferably in the range of 10 to 20 mm$^2$/s. If the kinematic viscosity is 5 mm$^2$/s or greater, the hydrocarbon oil (B) will be less able to volatilize off during production of the gel composition and variation in the composition of the gel composition will be less likely to occur, and therefore the physical properties of the gel after application onto the top sheet will be less likely to vary. If the kinematic viscosity is 50 mm$^2$/s or lower, the gel will be less likely to become hardened after application onto the top sheet, and a tacky property will be less likely to be produced. The kinematic viscosity of the hydrocarbon oil (B) can be determined by measurement according to JIS K 2283:2000, "5. Kinematic Viscosity Test Method", using a Cannon-Fenske reverse-flow viscometer, at a testing temperature of 37.8°C.

The content of the hydrocarbon oil (B) is in the range of 500 to 4800 parts by mass, preferably in the range of 1500 to 4500 parts by mass and even more preferably in the range of 2500 to 4000 parts by mass, with respect to 100 parts by mass of the styrene-based thermoplastic elastomer (A). If the content is 500 parts by mass or greater, the gel will be less likely to become hard and have reduced elongation after application onto the top sheet, and it will be possible to satisfactorily maintain the flexibility of the top sheet. If the content is 4800 parts by mass or lower, the gel will not become too soft, and therefore after application onto the skin side surface of the top sheet, the gel will be less likely to permeate too far to the non-skin side of the top sheet.

According to the invention, the gel composition further comprises an optional lubricant such as silicone oil, in addition to the hydrocarbon oil (B). The content of the optional lubricant is preferably in the range of 20 to 60 parts by mass with respect to 100 parts by mass of the styrene-based thermoplastic elastomer (A), from the viewpoint of sustained-release of the lubricant component.

In addition, the gel composition may comprise optional additives or other components, depending on the properties desired for the product. Examples of additives include stabilizers, antioxidants (for example, BHT (2,6-di-t-butyl-p-cresol), BHA (butylated hydroxyanisole), propyl gallate or the like), light stabilizers, coloring agents, pigments (for example, titanium oxide, zinc oxide or the like), aromatics, inorganic powders (for example, alumina, talc, mica, calcium carbonate, clay or the like), and organic powders (for example, PE, PP, silicone resin powder or the like). Examples of other components include oils with skin care functions (for example, jojoba oil or camellia oil), vitamins, various amino acids, peptides, zeolite, cholesterol, hyaluronic acid, lecithin, ceramide, skin astringents, anti-pimple medications, anti-wrinkle agents, anti-cellulite agents, skin whiteners, antimicrobial agents, antifungal agents, antiinflammatory components, pH regulators, humectants and the like.

According to the invention, the gel composition preferably has the specific viscosity characteristic of a viscosity of 100 to 500 mPa·s at 37°C. Since the gel composition can be coated at relatively low temperature when the gel composition has this specific viscosity characteristic, the components in the gel composition are less likely to undergo deterioration or volatilization (i.e., the composition of the gel composition is less likely to vary), and the function and effect of the gel composition can be stably exhibited. In addition, such a gel composition easily maintains its state immediately after coating, and changes in the dispersed state coefficient (V), i.e. changes in the dispersed state (especially changes in the size of the granular gel composition) that accompany temperature changes after coating are less likely to occur. As a result, it is possible to more reliably exhibit the aforementioned function and effect by which an excellent absorption rate and liquid repellent property can be obtained. Other advantages are that the gel composition can be coated at low temperatures, and that volatilization of the hydrocarbon oil and other components in the gel composition is less likely to occur, and therefore local aggregation of the elastomer components in the gel composition can be minimized and uniform coating can be carried out without clogging of the coating means by the gel composition. Incidentally, the viscosity of the gel composition may be measured using any desired viscometer (for example, an E-type viscometer).

The gel composition to be used in the top sheet of the invention can be produced by mixing each of the aforementioned formulating components using any desired mixing means, and for example, it can be produced by feeding the aforementioned formulating components either simultaneously or in an arbitrary order into a mixer, and melt mixing them in the mixer. The mixing means is not particularly restricted, and for example, a mixing apparatus such as a single-screw extruder, twin-screw extruder, roll, Banbury mixer, any of various types of kneaders, or a mixing kiln, may be used.

The top sheet of the invention can be obtained by coating the aforementioned hydrophobic gel composition onto one side of a nonwoven fabric that is composed of hydrophilic staple fibers (i.e., the skin side surface), under specific coating conditions for a dispersed state coefficient (V) of 900 to 5000.

The method of coating the gel composition will now be explained in detail.

[Method of coating gel composition]

The method of coating the gel composition for the top sheet of the invention is not particularly restricted so long as the dispersed state described above (i.e., a dispersed state with the dispersed state coefficient (V) in the range of 900 to 5000) can be obtained, and any desired coating means may be employed. Examples of such coating means include non-contact coaters such as spray coaters and curtain coaters, and non-contact coating means such as non-contact hot-melt applicators used for coating of hot-melt adhesives. Such non-contact coating means can render the gel composition into fine granular particles and uniformly disperse it on nonwoven fabric surfaces. Such non-contact coating means, incidentally, are advantageous in that they perform coating without contacting the discharge port with the nonwoven fabric surface, and therefore minimize clogging of the discharge port. Among such coating means, a non-contact hot-melt applicator is most preferably used because it comprises a discharge port through which the substance to be coated (hereunder referred to as "coating material") is to be discharged, and an air blowing port situated surrounding the discharge port, for blasting of air (pressurized air) onto the coating material to be discharged from the discharge port, allowing the coating material to be coated as a fine granular mist onto the article to be coated such as a nonwoven fabric, and facilitating adjustment of the coating conditions so that the dispersed state described above is obtained.

When the gel composition is to be coated using non-contact coating means, the coating temperature, the coating pressure and the coating amount are adjusted according to the viscosity of the gel composition used, so that the dispersed state described above is obtained. According to the invention, the coating temperature of the gel composition is usually in the range of 100°C to 180°C, preferably in the range of 110°C to 160°C and more preferably in the range of 120°C to 150°C. If the coating temperature of the gel composition is within this range, it will be possible to ensure the flow property while obtaining suitable atomization of the gel composition, so that the dispersed state described above can be easily obtained.

According to the invention, the coating pressure (air pressure) for the gel composition is preferably in the range of 0.001 MPa to 0.100 MPa and more preferably in the range of 0.01 MPa to 0.04 MPa. If the pressure is 0.001 MPa or higher, it will be possible to obtain suitable atomization of the gel composition and to diffuse the atomized gel composition over a wide area, such that clumping of the gel composition will be less likely to occur, and the fine granular gel composition can be uniformly coated in an evenly distributed manner. If the pressure is 0.100 MPa or lower, on the other hand, the gel composition will be able to reliably adhere onto the skin side surface of the top sheet and the interior of the top sheet, and the dispersed state of the gel composition will be unlikely to become too uniform (a state with a dispersed state coefficient (V) of smaller than 900), thereby helping to adequately ensure sections of the skin side surface of the top sheet through which excreted fluid such as urine permeates.

According to the invention, the coating amount of the gel composition (i.e., the basis weight of the gel composition in the dispersing region) will usually be in the range of 1 to 50 $g/m^2$, and is preferably in the range of 3 to 30 $g/m^2$ and more preferably in the range of 5 to 20 $g/m^2$. If the coating amount of the gel composition is 1 $g/m^2$ or greater, the hydrophobic action of the gel composition on the skin side surface of the top sheet (the action of pushing in excreted fluid) can be adequately exhibited, and therefore excreted fluid such as urine can be pushed into the top sheet in a steady manner and the absorption rate and liquid repellent property can be improved in a steady manner. Also if the coating amount of the gel composition is 50 $g/m^2$ or lower, excreted fluid supplied onto the skin side surface of the top sheet will not be repelled by the hydrophobic action of the gel composition but will be able to be stably pushed into the top sheet, and therefore the absorption rate and liquid repellent property can be exhibited in an even more steady manner.

For the purpose of the present description, the coating amount of the gel composition (the basis weight of the gel composition in the dispersing region) may be determined in the following manner.

(1) A prescribed region of the gel composition-coated top sheet that is to be measured is cut out using a sharp blade such as a cutter replacement blade, while avoiding any alteration in thickness, to obtain a sample for measurement of the coating amount.
(2) The area of the cut out sample: SA ($m^2$) and the mass: $SM_0$ (g) are measured.
(3) The measured sample is dipped in a solvent in which the gel composition is soluble, such as an aromatic solvent (for example, toluene) and stirred for at least 3 minutes for elution of the gel composition into the solvent.
(4) The sample in the solvent is filtered using mass-measured filter paper, and the sample is thoroughly rinsed with the solvent on the filter paper. Each rinsed sample filter paper sheet is thoroughly dried in an oven at 100°C.
(5) The masses of the dried filter paper and the sample are measured, and the pre-measured mass of the filter paper is subtracted from that value to calculate the dry sample mass: $SM_1$ (g).
(6) The gel composition coating amount $G_{BS}$ ($g/m^2$) is calculated by the following formula (1).
[Mathematical Formula 1]

$$G_{BS} \ (g / m^2) = [SM_0 \ (g) - SM_1 \ (g)] / SA \ (m^2) \qquad (1)$$

In order to reduce measurement error for the gel composition coating amount, multiple samples are cut out from multiple absorbent articles, so that the total area of the sample exceeds 100 $cm^2$, and each sample is measured according to (2) to (6) above, taking the average value for the coating amount $G_{BS}$ obtained from the measuring operations.

For a top sheet 2 according to one embodiment of the invention, the gel composition is coated onto the surface of a nonwoven fabric composed of staple fibers F (the skin side surface 21 of the top sheet 2) with the non-contact hot-melt applicator mentioned above, at a prescribed coating temperature, coating pressure and coating amount, and adheres to the staple fibers F of the nonwoven fabric in a dispersed state in which the dispersed state coefficient (V) is in the range of 900 to 5000 in the gel composition coated region (dispersing region). As shown in Fig. 2, in such a top sheet 2, a portion of the granular gel composition G infiltrates to the depth of the top sheet 2 and further, and therefore the hydrophobic action of the hydrophobic gel composition (i.e., the action of pushing excreted fluid such as urine to the non-skin side of the top sheet 2) also acts on the interior of the top sheet 2, making it possible to form a state in which excreted fluid such as urine is easily drawn in even deeper than the interior of the top sheet (the non-skin side) or to the non-skin side surface 22. As a result, the top sheet 2 can exhibit an excellent absorption rate and liquid repellent property (especially an excellent liquid repellent property).

According to the invention, the region in which the gel composition is to be coated on the top sheet (i.e., the proportion of the area of the coated region (dispersing region) with respect to the total area on the skin side surface of the top sheet) is preferably an area ratio of about 1% to about 90%, more preferably an area ratio of about 5% to about 60% and most preferably an area ratio of about 10% to about 30%, with respect to the area on the skin side surface of the top sheet (hereunder referred to simply as "top sheet area"). If the gel composition is coated at an area ratio of at least 1% of the total area of the skin side surface of the top sheet, the dispersing region of the gel composition will be wide and it will be possible to adequately ensure hydrophobicity on the skin side surface of the top sheet, and consequently the aforementioned excellent absorption rate and liquid repellent property will be easily obtained, while the gel composition will be present in at least a fixed amount on the top sheet, and the function and effect of the gel composition itself will also be adequately exhibited. On the other hand, if the gel composition is coated to an area ratio not exceeding 90% of the total area of the skin side surface of the top sheet, it will be possible to ensure a fixed minimum of regions of the top sheet that are not coated with the gel composition (i.e. the non-coated regions), so that permeation of excreted fluid that has been supplied to the skin side surface of the top sheet into the top sheet will be less likely to be inhibited.

The gel composition in the top sheet of the invention may be coated onto the top sheet in the manufacturing line for the absorbent article, or it may be coated onto the top sheet in a further downstream step of the manufacturing line (for example, just before the step of individual packaging of the product.

As shown in Fig. 1, the top sheet 2 according to one embodiment of the invention is a liquid-permeable nonwoven fabric situated on the skin side in the thickness direction $D_T$ of the disposable diaper 1, at the center axis line $C_L$ running in the lengthwise direction $D_L$ of the disposable diaper 1, and it is capable of direct contact with the skin surface of the wearer.

The nonwoven fabric used to compose the top sheet of the invention will now be described in detail.

[Nonwoven fabric]

The nonwoven fabric composing the top sheet 2 of this embodiment is not particularly restricted so long as it is composed of staple fibers, and for example, any desired staple fiber nonwoven fabric may be used such as an air-through nonwoven fabric or spunlace nonwoven fabric, or a wetlaid nonwoven fabric. There are no particular restrictions on the fiber lengths of the staple fibers composing such a staple fiber nonwoven fabric, but from the viewpoint of feel on the skin, and liquid permeability, flexibility, air permeability, bulk and strength of the nonwoven fabric, they are preferably 20 mm to 80 mm and more preferably 35 mm to 65 mm. Incidentally, because the top sheet on which the gel composition is coated according to the invention is a nonwoven fabric composed of staple fibers, the fine granular gel composition discharged from the aforementioned non-contact coating means readily infiltrates between the staple fibers composing the nonwoven fabric, and some of the granular gel composition in the gel composition coated region (dispersing region) even infiltrates to the interior of the top sheet. Moreover, since excreted fluid such as urine tends to be pushed even deeper than the interior of the top sheet (to the non-skin side), due to the hydrophobic action of the granular gel composition that has infiltrated the interior (the action of pushing in the excreted fluid), the aforementioned excellent absorption rate and liquid repellent property (liquid repelling rate) can be even more effectively exhibited.

The staple fibers composing the nonwoven fabric are not particularly restricted so long as they are hydrophilic, and they may be natural fibers or chemical fibers, for example, and more specifically, cellulose fibers such as ground pulp or cotton; regenerated cellulose such as rayon; semi-synthetic cellulose such as acetate; heat sealable composite fibers; or hydrophilicized thermoplastic hydrophobic chemical fibers. Also, examples of thermoplastic hydrophobic chemical fibers include synthetic fibers made of polyethylene (PE), polypropylene (PP) and polyethylene terephthalate (PET). The nonwoven fabric may be subjected to hydrophilicizing treatment after formation of the nonwoven fabric. According to the invention, the staple fibers composing the nonwoven fabric are most preferably fibers formed of a hydrophilic material, rather than hydrophilicized fibers.

Furthermore, the fiber size of the fibers composing the nonwoven fabric is not particularly restricted, but from the viewpoint of feel on the skin, liquid permeability, air permeability, bulk and strength of the nonwoven fabric, it is preferably 0.5 dtex to 10.0 dtex and more preferably 1.0 dtex to 5.0 dtex. The fiber size can be determined by magnified observation of the fibers using a scanning electron microscope or the like.

As shown in Fig. 1, the nonwoven fabric in the top sheet 2 of this embodiment has an essentially rectangular outer shape with the lengthwise direction $D_L$ as the long sides in the planar view; however, the top sheet of the invention is not limited to such a shape, and the nonwoven fabric may be in a quadrilateral shape other than rectangular, or it may include curves, such as an ellipsoid, elliptical or gourd shape. Moreover, the nonwoven fabric in the top sheet of the invention may be a nonwoven fabric with a monolayer structure composed of staple fibers, or a nonwoven fabric having a layered structure of two or more layers with other nonwoven fabrics stacked on the non-skin side of the nonwoven fabric composed of staple fibers.

Moreover, the size of the top sheet is not particularly restricted so long as it is a size that can cover the entire surface on the skin side of the absorbent body that is situated on the non-skin side of the top sheet, and any desired size may be employed, depending on the size and gender of the intended wearer of the absorbent article, and the purpose of use. The thickness of the top sheet may be any desired thickness in consideration of the liquid permeability, strength, flexibility and feel during wear. For example, the thickness of the top sheet may generally be in the range of 0.001 mm to 5.0 mm; however, from the viewpoint of suitable liquid permeability, cushioning properties and feel on the skin, it is preferably 0.01 mm to 3.0 mm and more preferably 0.1 mm to 1.0 mm.

For this embodiment of the invention, the top sheet 2 has a flat structure as shown in Fig. 1 and Fig. 2; however, there is no limitation to such a structure for the top sheet of the invention. For example, the top sheet used is preferably one having a structure with either or both a plurality of raised sections and a plurality of recesses on at least the skin side surface. When the top sheet used has a plurality of raised sections on the skin side surface, preferably at least some of the raised sections among the plurality of raised sections are coated with the gel composition. The plurality of raised sections are the sections that may directly contact with the skin of the wearer, and when these sections are coated with the gel composition they become hydrophobic, thereby inhibiting excreted fluid such as urine from pooling in or rewetting those sections, so that it is possible to prevent adhesion of excreted fluid such as urine onto the wearer. As a result, it is possible to provide the wearer of the absorbent article with an excellent smooth feel during wear.

In addition, when the top sheet has a plurality of raised sections on the skin side surface, the raised sections used may have any desired structures depending on the desired absorption rate, liquid repellent property, cushioning property and feel on the skin. For example, the raised sections may be ridges (elevations) extending in the lengthwise direction of the top sheet, or they may be protrusions with three-dimensional shapes, such as flat rectangular solid or truncated square pyramids having rounded edge lines, pyramidal shapes (for example, triangular pyramids or square pyramids) having rounded apexes, or conical or arched shapes having rounded apexes. The structures at the sections other than the raised sections mentioned above are not particularly restricted and may be either furrowed or flat sections, and for example, in a top sheet according to another embodiment of the invention, a structure having either or both the aforementioned plurality of raised sections and plurality of recesses is a structure having a plurality of ridges and a plurality of furrows (a "ridge-furrow structure"). An example of such a ridge-furrow structure is a structure in which a plurality of ridges are formed as a plurality of straight linear ridges extending in parallel along the lengthwise direction of the top sheet and aligned at essentially equal intervals in the widthwise direction $D_W$, and with the sections between every two adjacent ridges formed as furrows running along the lengthwise direction of the top sheet.

In an absorbent article employing a top sheet having such a ridge-furrow structure, excreted fluid such as urine supplied onto the top sheet readily diffuses in the lengthwise direction $D_L$ of the absorbent article along the ridges and furrows, and it is therefore possible to cause excreted fluid to be diffused in the lengthwise direction $D_L$ while being absorbed from the dispersing region of the gel composition to the interior of the top sheet. Since this allows excreted fluid that has penetrated the top sheet to be absorbed through a wider region of the absorbent body, the absorption efficiency of the excreted fluid can be drastically improved. Incidentally, since an absorbent article employing a top sheet having such a ridge-furrow structure has minimal spread of excreted fluid in the widthwise direction $D_W$ of the absorbent article, it is possible to prevent leakage of excreted fluid in the widthwise direction $D_W$.

The method of forming the ridge-furrow structure on the top sheet is not particularly restricted, and it may be known method such as a method of forming a ridge-furrow structure by continuously blasting gas (usually air) onto the fiber web as disclosed in Japanese Unexamined Patent Publication No. 2008-25079, Japanese Unexamined Patent Publication No. 2008-23326 or Japanese Unexamined Patent Publication No. 2009-30218, for example, a method of utilizing vacuum forming, a method utilizing gear stretching, or a method utilizing heat extension of heat-extendable fibers and/or heat shrinkage of heat-shrinkable fibers.

The basis weight of the top sheet in the absorbent article of the invention is not particularly restricted and may be any desired basis weight in consideration of the liquid permeability, strength and flexibility. Examples of such a basis weight is a basis weight in the range of 10 g/m² to 100 g/m², with the range of 20 g/m² to 50 g/m² being preferred. If the basis weight is 10 g/m² or greater, satisfactory liquid permeability and surface strength as a top sheet will be obtained. If the basis weight is 100 g/m² or lower, satisfactory liquid permeability as a top sheet will be maintained, while stiffness will be unlikely to result and the wearer of the absorbent article will be less likely to experience unpleasantness or discomfort.

Other structural members of the absorbent article in which the top sheet of the invention is employed will now be described.

As shown in Fig. 1 and Fig. 2, in the disposable diaper 1, the absorbent body 4 is situated on the non-skin side of the top sheet 2, and it absorbs and retains excreted fluid such as urine that has permeated the top sheet 2. It is generally preferred to use an absorbent body that is bulky and resistant to deformation and has low chemical irritation, in consideration of water absorption and comfort during wear. From this viewpoint, the absorbent body 4 used is preferably one comprising an absorbent core 41, for absorption and retention of excreted fluid, and a core wrap sheet 42 enclosing at least the skin side surface of the absorbent core 41, as shown in Fig. 2. For this embodiment, the top sheet 2 and core wrap sheet 42 situated on the skin side of the absorbent body 4 are at least partially joined with any desired adhesive, such as a hot-melt adhesive.

The absorbent core used may be a composite material containing a fiber material such as fluff pulp; an airlaid nonwoven fabric, hydrophilic fibers such as cellulosic fibers; or hydrophilicized thermoplastic fibers, and a superabsorbent polymer such as sodium acrylate copolymer. The absorbent core does not have to contain a superabsorbent polymer, and for example, a core wrap sheet enclosing only the fiber material mentioned above may be used as the absorbent body.

Also, the core wrap sheet is not particularly restricted so long as it has sufficient liquid permeability to allow permeation of excreted fluid such as urine and a sufficient barrier property to prevent permeation of the enclosed absorbent core components (i.e., to prevent leakage of the fiber material composing the absorbent core), and examples that may be used include sheet-like fiber structures such as nonwoven fabrics, woven fabrics and knitted fabrics made of natural fibers or chemical fibers. More specifically, tissues of basis weight approximately 10 g/m$^2$ to approximately 30 g/m$^2$, liquid-permeable nonwoven fabrics and hydrophilic nonwoven fabrics may be suitably used.

The structure of the absorbent body is not particularly restricted, and any structure may be employed according to the desired absorption performance and size, and the purpose of use.

The liquid-impermeable back sheet 3 is also not particularly restricted, and any desired liquid-impermeable sheet member may be used, examples of such sheet members including air-permeable resin films comprising PE and PP, multilayer articles having air-permeable resin films attached to spunbond or spunlace nonwoven fabrics, and multilayer nonwoven fabrics such as SMS.

The absorbent article in which the top sheet of the invention is used may further contain any desired structural members, depending on the desired product properties and the purpose of use. Incidentally, the invention may be applied to any of various types of absorbent articles, such as a pants-type disposable diaper according to the embodiment described above, or a sanitary napkin, tape-type disposable diaper or incontinence pad, for example. The absorbent article of the invention is not restricted to the embodiments described above and the examples described below, and can incorporate appropriate combinations and modifications within a range that is not outside of the object and gist of the invention.

Examples

The invention will now be explained in greater detail using examples and comparative examples, with the understanding that the invention is not limited only to the examples.

Example 1

A gel composition comprising 2.5 mass% of a styrene-ethylene/propylene block copolymer (SEP) and 97.5 mass% of PARLEAM 6 (product of NOF Corp., branched chain hydrocarbon produced by copolymerization of liquid isoparaffin, isobutene and n-butene followed by addition of hydrogen, polymerization degree: approximately 5-10, weight-average molecular weight: approximately 330) was coated as a mist onto a top sheet composed of a nonwoven fabric with a 3 mm-pitch ridge-furrow structure, using a non-contact hot-melt applicator, under coating conditions with a coating temperature of 120°C and a coating pressure of 0.005 MPa and a coating amount of 12 g/m$^2$, to fabricate a top sheet sample having a granular gel composition adhering to the surface. The prepared top sheet sample was attached using a hot-melt adhesive onto the section of a commercially available infant paper diaper (Moony "Air-Fit" S-size, by Unicharm Corp.) from which the top sheet had been removed, to fabricate an absorbent article for Example 1.

Example 2

An absorbent article for Example 2 was fabricated in the same manner as Example 1, except that the coating pressure during coating of the gel composition on the top sheet was 0.01 MPa.

Example 3

An absorbent article for Example 3 was fabricated in the same manner as Example 1, except that the coating pressure during coating of the gel composition on the top sheet was 0.02 MPa.

Comparative Example 1

An absorbent article for Comparative Example 1 was fabricated in the same manner as Example 1, except that the gel composition was coated in a striped fashion onto the top sheet using a contact coater, with a coating amount of 12 g/m$^2$.

Comparative Example 2

An absorbent article for Comparative Example 2 was fabricated in the same manner as Example 1, except that the coating pressure during coating of the gel composition on the top sheet was 0.0025 MPa.

Comparative Example 3

An absorbent article for Comparative Example 3 was fabricated in the same manner as Example 1, except that the coating pressure during coating of the gel composition on the top sheet was 0.03 MPa.

Comparative Example 4

An absorbent article for Comparative Example 4 was fabricated in the same manner as Example 1, except that the coating pressure during coating of the gel composition on the top sheet was 0.04 MPa.

Comparative Example 5

An absorbent article for Comparative Example 5 was fabricated in the same manner as Example 1, except that the coating pressure during coating of the gel composition on the top sheet was 0.05 MPa.

The fabricated absorbent articles of Examples 1 to 3 and Comparative Examples 1 to 5 were measured to determine the variation, black value and dispersed state coefficient (V) of the granular gel composition, and the absorption rate and liquid repelling rate, according to [Method of measuring dispersed state coefficient (V)] and [Absorption property evaluation test] above. The measured values and images of the dispersed states are shown in Table 1.

[Table 1]

| | | | Example 1 | Example 2 | Example 3 | Comp. Example 1 | Comp. Example 2 | Comp. Example 3 | Comp. Example 4 | Comp. Example 5 |
|---|---|---|---|---|---|---|---|---|---|---|
| Coating conditions | Coated form | | Dispersed | Dispersed | Dispersed | Striped | Dispersed | Dispersed | Dispersed | Dispersed |
| | Coating pressure (MPa) | | 0.005 | 0.01 | 0.02 | - | 0.0025 | 0.03 | 0.04 | 0.06 |
| | Coating amount (g/m$^2$) | | 12 | 12 | 12 | 12 | 12 | 12 | 12 | 12 |
| Dispersed state | Skin side surface | Photographed image | | | | | | | | |
| | | Variation | 31.6 | 28.0 | 26.0 | - | 46.8 | 22.8 | 24.5 | 24.6 |
| | | Black value | 58.8 | 42.7 | 36.5 | - | 135.7 | 35.2 | 34.7 | 30.0 |
| | | Dispersed state coefficient (V) | 1860 | 1197 | 949 | - | 6349 | 802 | 851 | 741 |
| | Non-skin side surface | Photographed image | | | | | | | | |
| | | Variation | 40.1 | 40.7 | 43.3 | - | 38.7 | 41.4 | 41.6 | 39.1 |
| | | Black value | 120.4 | 117.3 | 96.7 | - | 170.9 | 101.9 | 94.1 | 87.4 |
| | | Dispersed state coefficient (V) | 4830 | 4780 | 4191 | - | 6614 | 4213 | 3915 | 3418 |

EP 3 318 235 A1

(continued)

| Absorption property evaluation | | Example 1 | Example 2 | Example 3 | Comp. Example 1 | Comp. Example 2 | Comp. Example 3 | Comp. Example 4 | Comp. Example 5 |
|---|---|---|---|---|---|---|---|---|---|
| | Absorption rate (sec) | 11.0 | 11.7 | 11.3 | 11.9 | 10.9 | 12.9 | 16.8 | 20.1 |
| | Liquid repelling rate (sec) | 48.5 | 45.3 | 43.3 | 54.4 | 61.7 | 46.7 | 53.2 | 67.0 |

As shown in Table 1, the absorbent articles of Examples 1 to 3 had more excellent absorption properties in terms of both absorption rate and liquid repelling rate, and an especially excellent liquid repelling rate, compared to the absorbent article of Comparative Example 1 that was coated in a striped fashion, and compared to the absorbent articles of Comparative Examples 2 to 5 in which the dispersed state coefficients (V) were outside of the range of 900 to 5000.

Reference Sign List

1 Disposable diaper (absorbent article)
2 Top sheet
3 Back sheet
4 Absorbent body
41 Absorbent core
42 Core wrap sheet
5 Side sheet
6 Dispersing region

**Claims**

1. A top sheet for an absorbent article,

   wherein the top sheet comprises a nonwoven fabric composed of staple fibers and a hydrophobic granular gel composition adhering to the staple fibers, and the skin side surface of the top sheet has a dispersing region in which the granular gel composition is present in a dispersed state, and
   the dispersed state coefficient (V), which represents the dispersed state of the granular gel composition, is 900 to 5000 in the dispersing region.

2. The top sheet according to claim 1, wherein the dispersed state coefficient (V) in a region corresponding to the dispersing region on a non-skin side surface is 4000 to 6000.

3. The top sheet according to claim 1 or 2, wherein the gel composition has a viscosity of 100 to 500 mPa·s at 37°C.

4. The top sheet according to any one of claims 1 to 3, wherein the gel composition is present at a basis weight of 1 to 50 $g/m^2$ in the dispersing region.

5. The top sheet according to any one of claims 1 to 4, wherein the gel composition is a composition comprising a styrene-based thermoplastic elastomer that forms a gel network structure, and a hydrocarbon oil.

6. The top sheet according to claim 5, wherein the styrene-based thermoplastic elastomer is a styrene-based thermoplastic elastomer composed of a diblock copolymer.

7. The top sheet according to any one of claims 1 to 6, wherein the gel composition is a composition comprising 100 parts by mass of styrene-based thermoplastic elastomer (A) composed of a diblock copolymer with a weight-average molecular weight of from 100,000 to 200,000, and 500 to 4800 parts by mass of a hydrocarbon oil (B) with a kinematic viscosity of 5 to 50 $mm^2/s$ at 37.8°C.

8. An absorbent article including a top sheet according to any one of claims 1 to 7.

# FIG. 1

# FIG. 2

## INTERNATIONAL SEARCH REPORT

| International application No. |
| --- |
| PCT/JP2016/065207 |

| A. CLASSIFICATION OF SUBJECT MATTER |
| --- |
| A61F13/511(2006.01)i |

According to International Patent Classification (IPC) or to both national classification and IPC

| B. FIELDS SEARCHED |
| --- |
| Minimum documentation searched (classification system followed by classification symbols) |
| A61F13/15-13/84 |

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)

| C. DOCUMENTS CONSIDERED TO BE RELEVANT | | |
| --- | --- | --- |
| Category* | Citation of document, with indication, where appropriate, of the relevant passages | Relevant to claim No. |
| A | JP 5677611 B1 (Uni-Charm Corp.), 25 February 2015 (25.02.2015), & WO 2016/002092 A1 & CN 105407850 A | 1-8 |
| A | JP 2008-522772 A (The Procter & Gamble Co.), 03 July 2008 (03.07.2008), & US 2006/0135920 A1 & WO 2006/066107 A1 & EP 1671609 A1 & CN 101080209 A | 1-8 |
| A | JP 2013-212250 A (Uni-Charm Corp.), 17 October 2013 (17.10.2013), & US 2015/0080838 A1 & WO 2013/150921 A1 & EP 2835120 A1 & CN 104334137 A | 1-8 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 01 August 2016 (01.08.16) | 09 August 2016 (09.08.16) |

| Name and mailing address of the ISA/ Japan Patent Office 3-4-3,Kasumigaseki,Chiyoda-ku, Tokyo 100-8915,Japan | Authorized officer |
| --- | --- |
| | Telephone No. |

Form PCT/ISA/210 (second sheet) (January 2015)

**INTERNATIONAL SEARCH REPORT**

International application No.

PCT/JP2016/065207

C (Continuation). DOCUMENTS CONSIDERED TO BE RELEVANT

| Category* | Citation of document, with indication, where appropriate, of the relevant passages | Relevant to claim No. |
|---|---|---|
| A | JP 2008-529721 A (The Procter & Gamble Co.), 07 August 2008 (07.08.2008), & US 2006/0184150 A1 & WO 2006/088667 A1 & EP 1848392 A1 & KR 10-2007-0104604 A & CN 101119692 A | 1-8 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008522772 A
- JP 5677611 B
- JP 2008025079 A
- JP 2008023326 A
- JP 2009030218 A